# EUROPEAN PATENT APPLICATION

(11) **EP 0 873 718 A2**
(43) Date of publication of application: **28.10.1998**
(21) Application number: 98202590.0
(22) Date of filing: 19.04.1996
(51) Int. Cl.: A61B 17/064, A61B 17/70, A61F 2/44, A61B 17/68

(54) **Devices for osteosynthesis**

(30) Priority: 28.04.1995 RU 95107138; 27.09.1995 RU 95116522; 09.12.1995 RU 95121179
(62) Divisional of application: 96201055.9
(71) Applicant: Gazzani, Romolo Igino, 15069 Serravalle Scrivia (Alessandria) (IT); Shaboldo, Oleg Pavlovich, St. Petersburg (RU)
(72) Inventor: Davydov, Evgeni Alexandrovich, St. Petersburg (RU); Davydov, Denis Evgenievich, St. Petersburg (RU); Shaboldo, Oleg Pavlovich, St. Petersburg (RU); Novoselov, Konstantin Anatolievich, St. Petersburg (RU); Zasulski, Philipp Yurievich, St. Petersburg (RU)
(74) Representative: Martegani, Franco

(57) **Abstract**

A device for osteosynthesis is of the kind prepared of material which exhibits form-memory effect, is made of wire and contains a working part and locking components. According to the invention, the locking components are bent in either one or, parallel or perpendicular planes with at least one of them being a closed loop.

## Description

The invention is related to devices for ostesynthesis and can be used in traumatology, neurotraumatology, orthopedics, vertebrology, in case of traumas and surgical operations on the long tubular bones (including periathric regions), fractures of spine, vertebral dislocations, traumas and pathologies of other skeleton bones.

A number of plunge constructions have been suggested for use in surgery for the fixation of bone fragments: different kinds of plates, screws, wires, nails, etc.. See Williams D.Ph., Rouf R. Implantants in Surgery (in Russian), M.Medicine, 1978 pp.409-450 and in DE 4210801.

The majority of plunge constructions (especially those with compression mechanism) all share one main disadvantage: the effect of compression lasts only for several days, afterwards device is turning into a sort of "spacer", which leads to the instability of bone fragments that is, to violations of the reparative regeneration. See Devyatov A.A. Transosseous Osteosynthesis. Kishinev; Shtinica, 1990 p.314.

To avoid such complications, devices of a material displaying form-memory effect are being applied successfully ensuring constant bone fragments compression for the whole therapy period. See Gunter V.E., Kostenko V.V., Mirgazinov M.Z. et al. The Alloys with Form-Memory in Medicine. Tomsk; Tomsk University Ed., 1986, pp.116-119; and Gunter V.E., Kostenko V.V., Mirgazinov M.Z. et al. The Alloys with Form-Memory in Medicine. Tomsk; Tomsk University Ed., 1986, pp. 68-73. Depending on the type of the fracture (pathology), it is often necessary, aside from firm fixation of bone fragments, to ensure required compressing force or to substitute bone fragment with a prosthesis keeping the function of the missing fragment. In these cases the form of the implantant made of material with form-memory effect is crucial.

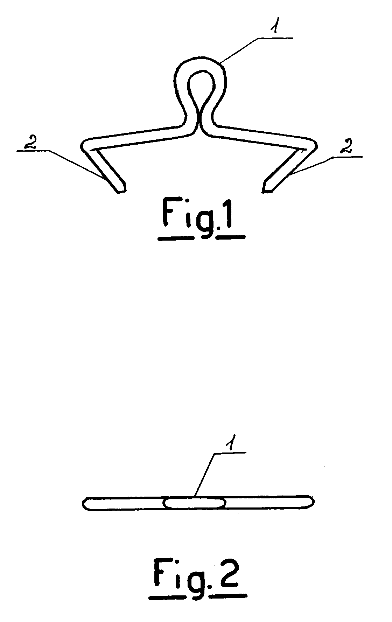

From the devices of alloys with form-memory effect taken into medical practice, the most convenient are wire constructions of titanium nikelide. One of these devices (Figures 1,2) is made in the shape of a clip and contains two tapered legs (2) and working part (1), the last consisting of one or several arched components (rings), positioned in the same or orthogonal planes relative to the legs. See Gunter V.E., Etin V.I., Monasevich L.I. et al. Form-Memory Effects and their Application in Medicine. Novosibirsk; Nauka, Sib. Branch., 1992, pp.502-518. Prototype.

Besides, the middle part of the device is made arch-shaped. To decrease mass and overall dimensions of the device keeping unchanged the developed force it is made of material with low temperature values of the phase transition.

This device of the prior art has the following disadvantages:
1. The developed force when clamping long tubular bone fragments is insufficient.
2. Inapplicable for treatment of the periathric fractures of long tubular bone fractures and joint pathologies (e.g., correcting osteotomy).
3. When shaping the device in the cooled state to a form convenient for the installation, it is necessary to deform its components separately, thus complicating surgical operation and increasing its duration.
4. When using a material with low temperature values of the phase transition in devices with small working rings diameter, destruction of the devices in the postoperative period is possible due to the overstrain of the material.
5. The device can be used in surgical operations on the spine with vertebral fractures and dislocation fractures. However, this may make vertebrae destruction possible because of the locking component (hook) action, which leads to the complications in the postoperative period. It also eliminates the use of the device in a number of cases, concerning fractures of different vertebral body parts (e.g. spinous process).
6. This device as well as related to it by the technical essence and attained results are made of alloys on the basis of titanium nikelide with low temperatures of the phase transition (T=-30...+35°C). These devices application in neurosurgery may lead to the form-memory effect occurring during the installation, which aside from the difficulties for the surgeon presents danger for the patient's life (injuries of the open spinal marrow regions are possible). Liquid nitrogen or chloroethyl utilisation for the device cooling also complicates the course of the operation.
7. The device is practically inapplicable in surgical operations on the spine connected with intervertebral disk removal, for it does not ensure recovery of the normal (initial) anatomic alignment of the vertebrae and the spine axis.

The general object of the present invention is to obviate the aforesaid disadvantages of the prior art.

This object is attained by devices for osteosynthesis having the features exposed in the annexed claims.

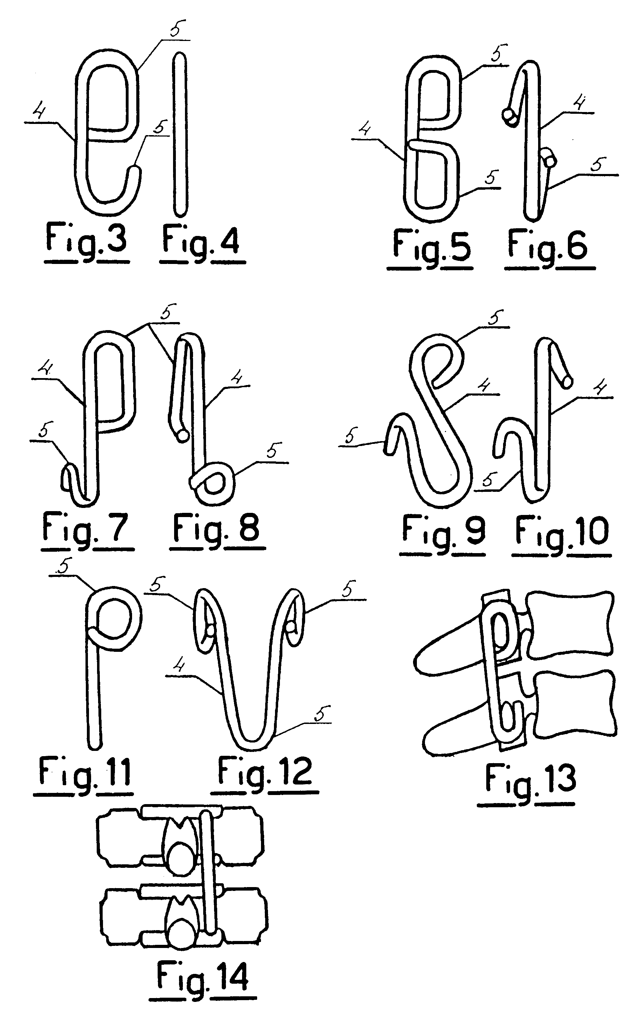

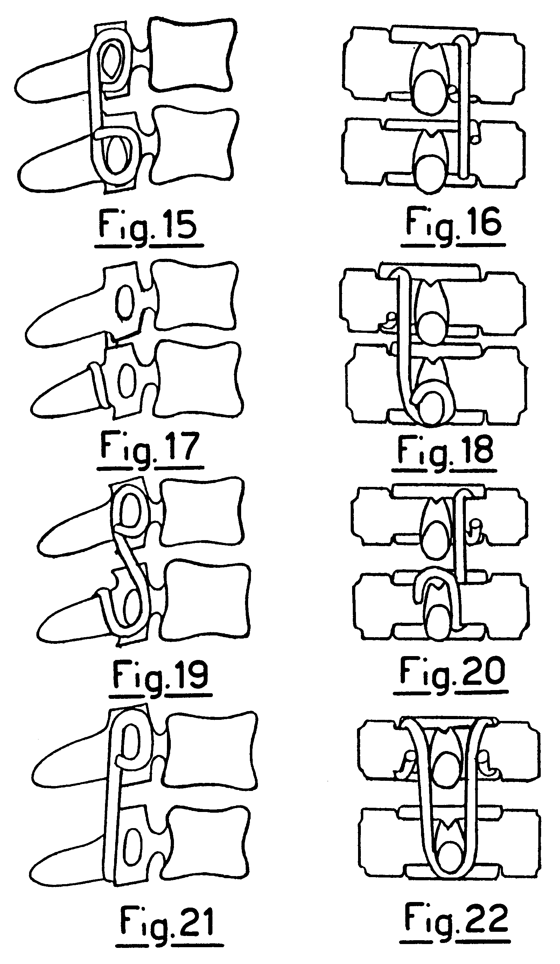

Figures 3-22 show embodiments of the invention.

Suggested solution according to the present invention is directed towards creating a set of the wire devices prepared from the material with form-memory effect and includes several versions.

Each of the proposed devices can be used separately during fracture therapy and surgical operations on the long tubular bones (including periarthric regions), in therapy of the spine fractures, vertebral dislocations, as well as in combinations with one another in course of more complicated operations.

To provide compression osteosynthesis, the device (of material, exhibiting form-memory effect) made of the wire and containing working part and locking components, is used.

Suggested device (versions) is represented in Fig.3-12 in the working state - side and back views. Fig.13-22 show the device (its versions) being installed on the spine - side and back views.

The device consists of the wire clip 4 and the locking components 5 with the latter positioned either in one (Fig.3,4) or parallel (Fig.5,6,11,12) or perpendicular (Fig.7-10) planes. The constructions shown are convenient for eliminating insignificant instability of the locomotory spine segment. These constructions can also be applied in more complicated cases in combinations with other versions of the device. They are easily installed on vertebral arches and spinous processes. Constructions (Fig.7-10) are used on more critical traumas and pathologies (including development pathology), e.g. rotatory subluxations in the locomotory segment, caries of an arch of the underlying vertebrae, etc. These devices are installed on the upper vertebra arch and the lower vertebra spinous process (Fig.17-20). Constructions (Fig.11,12) are employed on the bilateral subluxations in the locomotory segment. These are set up from both sides of the spine on the upper vertebra arch and put under the spinous process of the lower vertebra.

Making at least one of the locking components as a closed loop is aimed at ensuring reliable clamping of the device (on either vertebral archs or spinous processes).

Positioning locking components in the same, parallel or perpendicular planes allows the realisation of the firm vertebrae clamping with the whole variety of the spine traumas and pathologies.

The design of the device and the installation procedure are fairly easy and it does not take very much time for the surgeon to put it in (1-2 minutes is enough). This is the reason to use a material (exhibiting form-memory effect) with the phase transition top limit not greater than +45°C. At the meantime, the bottom limit has to be +30°C or higher to prevent premature spontaneous operating of the device. Indicated temperature values prevent recovery of the previously set form by the device when heated to the temperature of the operating room. The device made from such material does not require specific conditions for cooling and can be shaped at +10...+20°C.

The device is applied as follows: the inferior access to the required spine section is performed and depending on the trauma or pathology type needed version of the device (several versions at a time can also be used) is chosen and used separately or in combination with other known devices prepared from materials with form-memory effect.

The fixation is performed on either arches or arches with spinous processes (the components of the device are preliminary cooled down to +10°...+20°C and shaped to the form convenient to put it in). After the installation the device is heated up by warm physiological salt solution, locking components recover their initial form and firmly clamp the spine.

Use of the proposed device allows to achieve reliable postoperative stability by considering the specificity of traumas, pathologies and the individual organism structure of a patient as well as to completely recover the function of the injured spine segment, decrease traumaticity and duration (up to 20-30%) of the surgical operation.

To obtain the best results of the surgical treatment of the spine, it is necessary as soon as possible to eliminate the compression of the spinal marrow and to recover normal (initial) anatomical correlation of the vertebrae and the spine axis. In particular, operations on the vertebral unstable fractures or dislocation fractures makes necessary either:
i) intervertebral disk removal with the following firm clamping to achieve primary postoperative stability (until the osteofibrous block formation); or
ii) consolidation on the level of the injured locomotory spine segment.

Indicated therapy methods are put into practice by making device in the shape of a single-coil helix, which is made of the material with form-memory effect at +30...+45°C temperature range. At its ends the helix has tapered legs, directed to the opposite sides of the line parallel to the helix axis. This device serves as a fixator, being, as a matter of fact, an endoprosthesis of the intervertebral disk. It provides the decrease of traumaticity and surgical operation duration, prevents vertebrae from shifting, eliminates the danger of the spinal marrow compression and ensures maximal recovery of the anatomical spine structure and its functioning in the early postoperative period.

The proposed device is prepared of wire (1,5-4 mm in diameter) of the material exhibiting form-memory effect (e.g., titanium nikelide with slight ni excess: 50.2-50.5 at.%). To provide form-memory effect in the required (depending on the version and purpose) temperature range, constructions are subjected to thermal and thermomechanical treatment by different known procedures.

See Khachin V.M., Pushin V.G., Kondratyev V.V., Titanium Nikelide: Structure and Properties. M; Nauka, 1992, pp.143-146.

Application of the set of different versions of the device allows to obtain reliable clamping of the bone fragments and transplantants during operations on different skeleton sections in case of traumas and pathologies of various difficulty.

## Claims

1. The device for osteosynthesis is of the kind prepared of material which exhbits form-memory effect, is made of wire and contains a working part and locking components, and is characterised in that the locking components are bent in either one or parallel or perpendicular planes with at least one of them being a closed loop.

2. The device according to claim 1, characterised in that the material exhibits form-memory effect in +30...+45°C temperature range.
